# EUROPEAN PATENT APPLICATION

(11) **EP 3 954 342 A1**
(43) Date of publication of application: **16.02.2022**
(21) Application number: 21183850.3
(22) Date of filing: 06.07.2021
(51) Int. Cl.: A61F 5/01

(54) **DYNAMIC FINGER SPLINT**

(30) Priority: 12.08.2020 NL 2026259
(71) Applicant: WE Design Beheer B.V., 6814 EN Arnhem (NL)
(72) Inventor: ENGELSHOVEN, Wouter Robin, 6815 AK Arnhem (NL)
(74) Representative: Nederlandsch Octrooibureau

(57) **Abstract**

A dynamic finger splint for preventing over-stretching of both a proximal interphalangeal joint (PIP) and a distal interphalangeal joint (DIP) of a finger, said splint comprising a rigid distal ring splint a rigid proximal ring splint and a dorsal connector, hingeably connecting the proximal ring splint to the distal ring splint on the dorsal side, defining an axis of rotation substantially perpendicular to a symmetry plane of the finger splint wherein, the dorsal connector is formed by two loops, which are attached to the first dorsal support, each loop being positioned on an opposite side of said symmetry plane, and forming a closed loop around at least a part of a circumference of the third dorsal support, each loop having an inner diameter substantially corresponding to a width of the at least part of the circumference of the third dorsal support.

## Description

### Field of the invention

The present invention relates to a dynamic finger splint for preventing over-stretching of both a proximal interphalangeal joint (PIP) and a distal interphalangeal joint (DIP) of a finger, said splint comprising: a rigid distal ring splint which comprises a first proximal ring, sized to fit around an intermediate phalanx of the finger, having a first dorsal support for abutting an approximate longitudinal centre of the intermediate phalanx on a dorsal side with an inner surface and a section opposite thereof for abutting the intermediate phalanx at the distal interphalangeal joint on a palmar side with an inner surface, a first distal ring, sized to fit around a distal phalanx of the finger, having a second dorsal support for abutting an approximate longitudinal centre of the distal phalanx on the dorsal side with an inner surface and a section opposite thereof for abutting the distal phalanx at the distal interphalangeal joint on a palmar side with an inner surface, and a rigid distal palmar connection, connecting the two sections of the first proximal and distal rings, for abutting at the distal interphalangeal joint, at an angle with respect to one another and having a width, such that, when worn, the distal interphalangeal joint can be bent over the distal palmar connection; a rigid proximal ring splint which comprises a second distal ring, sized to fit around the intermediate phalanx of the finger, having a third dorsal support, adjoining the first proximal ring on the dorsal side and a section opposite thereof for abutting the intermediate phalanx at the proximal interphalangeal joint on a palmar side with an inner surface, a second proximal ring, sized to fit around the proximal phalanx of the finger, having a fourth dorsal support for abutting an approximate longitudinal centre of the proximal phalanx on the dorsal side with an inner surface and a section opposite thereof for abutting the proximal phalanx at the proximal interphalangeal joint on a palmar side, a rigid proximal palmar connection, connecting the two sections of the second proximal and distal rings for abutting the proximal interphalangeal joint, at an angle with respect to one another and having a cross-section, such that, when worn, the proximal interphalangeal joint can be bent over the proximal palmar connection; and a dorsal connector, hingeably connecting the proximal ring splint to the distal ring splint on the dorsal side, defining an axis of rotation substantially perpendicular to a symmetry plane of the finger splint. Further, the invention relates to a method of manufacturing such a finger splint.

### Background art

Known splints of this type have a metallic band which is attached to the first dorsal support of the first proximal ring and loops around the third dorsal support of the second distal ring, wherein said band is positioned in a symmetry plane on the dorsal side of the finger splint. The band fits tightly around the third dorsal support, with the third dorsal support and band being shaped such that the third dorsal support is rotatable to some degree around the axis of rotation extending through the loop which is defined by the band.

Each of the distal and proximal ring splints is shaped such that, when worn, the respective joint around which they are fitted can be flexed by at least some degree, while rotation of each joint toward the dorsal side of the finger is limited, such as to prevent over-stretching of both the PIP and the DIP. The axis of rotation provided by the hinging loop allows the distal ring splint being rotated with respect to the proximal ring splint, thereby enabling both PIP and DIP being flexed simultaneously, such as is required for performing a gripping motion with the finger. Due to the band being positioned onto the intermediate phalanx, at the location where the third dorsal support abuts the first dorsal support, the hinging motion allowed by the dorsal connector is automatically blocked once the finger around which the finger splint is worn is extended along a longitudinal axis through the finger splint.

A downside of this finger splint design is that the maximum deflection of the rigid distal and proximal ring splints with respect to one another is determined by the size of the rings, which depends on the measurements of a patient's fingers. As a result, the maximum deflection cannot be adjusted to a desired amount of freedom of movement. Instead, flexion between the rigid distal and proximal ring splints, towards the palmar side, is limited to approximately 25 degrees. Furthermore, the dorsal connector only provides reliable automatic blocking of rotation until the band suffers from wear, causing the third dorsal support no longer being tightly held against the first dorsal supprt. Once the dorsal connector starts to wear, over-stretching of the DIP is no longer fully prevented.

A further downside is that the finger splint is difficult to adjust to the exact anatomy of a patients finger once made. A distance between the distal and proximal palmar connections depends on the distance of the palmar connection to the first and third dorsal supports and an angle of rotation there between. The first and second dorsal and proximal rings are made according to a set of measurements and assembled. In case the assembled finger splint does not perfectly match the anatomy of a patient's finger during use, one or both of the ring splints moves with respect to the finger when the finger is flexed or stretched, such that one or both of the palmar connections is no longer aligned with the DIP and/or PIP, limit the freedom of flexing, effectivity of protecting from over-stretching and/or causing discomfort. Especially the distal rigid splint is known to move towards the finger tip,resulting in that the finger is forced to move alltogether by the splint when attempting to flexi the finger, causing a lot of discomfort and loss of hand function.

The US patent application US 2012/0289877 A1 discloses a phalangeal deformity ring splint, in particular a splint that includes a first loop, a second at least partial loop, an elongated support and optionally a third at least partial loop. The first loop and the second at least partial loops are attached to the elongated support such that the elongated support contacts the ventral surface of a phalange when in position on a phalange.

### Summary of the invention

The present invention aims to provide an improved dynamic finger splint, in particular a dynamic finger splint that is more durable and easier to make fitting to a patient's finger such that it is more comfortable to wear.

According to the present invention, a finger splint as mentioned in the preamble is provided, wherein the dorsal connector is formed by two loops, which are attached to the first dorsal support, each loop being positioned on an opposite side of said symmetry plane, and forming a closed loop around at least a part of a circumference of the third dorsal support, each loop having an inner diameter substantially corresponding to a width of the at least part of the circumference of the third dorsal support.

The first and second proximal and distal rings are sized such that they each have inner surface surrounding a circumference of the finger. The loops being spaced apart from one another around the plane of symmetry and looping around at least a part of the circumference of the third dorsal support, results in a distance being present between a centre of the third dorsal support, along the plane of symmetry, and the axis of rotation defined by the dorsal connector. This distance can be adjusted by adjusting the distance between the loops, resulting in a distance between the rigid distal palmar connections of the distal and proximal ring splints being adjustable independently from the shape of the distal and proximal ring splints.

Additionally, due to the loops being positioned on both sides of the symmetry plane, rather than in the symmetry plane, the axis of rotation formed thereby is closer to an axis extending through the PIP and DIP when the finger splint is worn. As a result, the finger does not limit the flexing rotation of the finger splint as much. Thus the finger splint according to the invention provides freedom of movement which is much closer to the freedom of movement of a normal, uninjured finger. Furthermore, due to the dorsal hinge comprising two loops on opposite sides of the symmetry plane, the position of the axis of rotation formed thereby with respect to the first dorsal support is more rigidly fixed. Forces acting on the distal joint, into the plane of symmetry result in a rotational force at the hinge, acting perpendicular to the axis of rotation, in the plane of symmetry. Having two loops forming the axis of rotation, spaced apart from one another on opposite sides of the plane of symmetry, enables the finger splint to successfully take up this rotational force. The dorsal connector in the finger splint according to the invention thus additionally provides bilateral support and protection to the finger, when the finger splint is worn. Although such support may be achieved to some extend by a wide loop at the plane of symmetry, the rotational force directly acts on the wide loop, making the loop more prone to wear and deformation, which immediately negatively affect the bilateral support provided by a finger splint with such a dorsal connector. As a result, the finger splint according to the invention not only provides improved protection against overstretch (hyperextension), but also protects from sideways movements of the joints. Thus the improved splint is also most usefull for patients who suffer from laxity of finger joints in all directions.

According to an embodiment, the third dorsal support is provided with two through-holes on opposite sides from the symmetry plane, each through-hole having a loop passing therethrough.

Allows relatively small loops being used, even when the dorsal supports have a circumference with a large width. Relatively small loops are desirable, since the loops are less deformable during use and result in a more compact finger splint, which is more practical in use.

Furthermore, the two through-holes through which the loops pass, ensure that an axis projected through the centres of the first and second dorsal supports and and axis projected through the centres of the third and fourth dorsal supports is maintained within the plane of symmetry throughout use of the finger splint, further ensuring consistent bilateral support provided by the finger splint.

According to an embodiment, a width of the circumference of the third dorsal support along the symmetry plane is larger than the inner diameter of each loop, for ensuring that a position of the third dorsal support is maintained between the loops even when the loops are not passing through through-holes in the third dorsal support.

According to an embodiment, the dorsal connector is adapted to enable a hinging movement between a stretched position, in which an axis through the second and fourth dorsal supports along the symmetry plane extends through the first dorsal support, and a flexed position, in which a distance between the proximal and distal palmar connections is smaller than in the stretched position. This ensures that, when a finger wearing the splint is in the stretched position, the third dorsal support abuts a surface of the first dorsal support facing away from the finger, effectively blocking further rotation of the distal phalanx with respect to the proximal phalanx towards the dorsal side of the hand.

According to an embodiment, the angle between the proximal and distal rings at the proximal palmar connection is between 85 and 95 degrees, preferably 90 degrees and wherein the angle between the proximal and distal rings at the distal palmar connection is between 45 and 90 degrees, preferably between 55 and 65 degrees. As a result, the support of each ring is approximately at the centre of the phalanx, while the opposite longitudinal end of said ring is at the joint, when worn. When the rings of each ring splint are perfectly fitted around the circumference of the finger, the 90 degree angle delimits the freedom of movement of the two phalanges joined by the joint to the stretched position when rotating the phalanges towards the dorsal side. The angle may be adjusted to be slightly larger or smaller, depending on specific movability requirements and/or finger shape of the patient. Due to the improved blocking provided by the dorsal connector, the angle between the proximal and distal rings at the distal palmar connection may be smaller than 90 or even 85 degrees, such that the proximal ring of the distal ring splint is positioned closer to perpendicular with respect to the longitudinal direction of the finger. Since a distance between the axis of rotation and the distal palmar connection depends on the dimensions of the patient's finger, the location of the loops of the dorsal connector and the angle between the proximal and distal rings, a second variable (i.e. the angle) is now also adjustable to tailor the finger splint such that the distance between the palmar and distal connections matches the finger's PIP and DIP bending lines on the palmar side of the finger throughout bending motions. In practice, the angle between the proximal and distal rings at the palmar connection being between 55 and 65 degrees will be optimal for most fingers.

According to an embodiment, a distance between the second palmar connection and a centre of the third dorsal support is larger than a distance between the second palmar connection and the axis of rotation. Thus the distal and proximal ring splints overlap one another on the dorsal side of the intermediate phalanx. The amount of overlap depends on the exact positioning of the loops of the dorsal connector and may be adjusted based on the length of a patient's finger to ensure the palmar connections remain aligned with the PIP and DIP throughout flexing and stretching movements of the finger. The overlapping sections of the distal and proximal ring splints delimit the hinging motion at the stretched position. Advantageously, this overlapping section is in between the loops of the dorsal connector, rather than directly below. As a result, the loops of the dorsal connector are not directly involved in the delimiting of the stretching motion, and thus suffer less wear therefrom.

According to an embodiment, the first, second and fourth dorsal support each have a width along the symmetry plane between 2 and 9 mm, preferably between 2.5 and 8 mm. The width can be chosen to meet a patient's preference. The minimum width matches the minimal ring cross-sectional width. The minimal ring cross-sectional width may be increased in order to ensure the ring splints are sufficiently rigid. The dorsal supports may be provided with a larger width, such that a pressure felt on the dorsal side of the finger is distributed over a larger area, in order to increase comfort.

According to an embodiment, the finger splint being made of one or more metallic materials, preferably at least one of a silver alloy, a gold alloy, a titanium alloy or stainless steel. Metallic materials are relatively easy to work with and provide the finger splint with sufficient rigidity and durability. To prevent allergic reactions from frequent and extended wearing of the finger splint, use of nickel-free metallic materials is required. Metallic materials commonly used for both aesthetic and hypoallergenic reasons include silver-alloys, gold alloys, titanium alloys and stainless steel. Silver alloys are most preferred, as they are sufficiently rigid to maintain their shape during every day use, whilst being relatively simple to work with and adjust as well as being affordable.

According to a second aspect of the invention, a method of manufacturing a dynamic finger splint is provided, which comprises the steps of:
- obtaining a patient's finger measurements, determining a length and a circumference of each of the proximal, intermediate and distal phalanges,
- producing according to those measurements the rigid distal ring splint, for preventing over-stretch of a distal interphalangeal joint of the finger, and the rigid proximal ring splint, for preventing over-stretch of a proximal interphalangeal joint of the finger, the rigid distal ring splint,
- joining the proximal ring splint to the distal ring splint by providing two loops, each having an inner diameter equal to or smaller than a width of the third dorsal support along the symmetry plane and closing each loop around at least part of the circumference of the third dorsal support.

According to an embodiment, the step of joining further comprises providing two through-holes in the third dorsal support, which are spaced apart on opposite sides from the symmetry plane having a distance there between matching a distance between the two protrusions for forming loops.

According to an embodiment, the step of producing the rigid distal ring splint comprises providing two protrusion for forming loops, which are attached to the first dorsal support, spaced apart on opposite sides from the symmetry plane, and the step of joining the proximal ring splint to the distal ring splint comprises shaping each of the two protrusions into a loop having an inner diameter equal to or smaller than a width of the third dorsal support along the symmetry plane.

According to an embodiment, the step of producing the rigid distal and proximal ring splints comprises:
- producing a wax-mould of the rigid distal ring splint and of the proximal ring splint according to the patient's finger measurements, and
- casting the rigid distal ring splint and the rigid proximal ring splint from a metallic material.
The loops or protrusions for forming loops may be cast together with the rigid distal ring splint or added in a further manufacturing step.

According to an alternative embodiment, the step of producing the rigid distal and proximal ring splints comprises three-dimensional printing of the rigid distal and proximal ring splints from a metallic material. Similarly, the loops or protrusions for forming loops may be printed together with the rigid distal ring splint or added in a further manufacturing step.

According to an embodiment, the metallic material comprises at least one of silver, gold, bronze or titanium. According to an embodiment, the closing of each loop comprises soldering the loop to be closed. This is a method commonly used by silver- and goldsmiths to produce small, rigid loops for hinges, and thus a proven technology. The soldering is usually a manual step which allows the final assembly and finishing of the distal and proximal ring splints being tailored to specific patient needs.

### Brief description of the drawings

The present invention will be discussed in more detail below, with reference to the attached drawings, in which:
Figure 1A shows a dorsal view of a prior art dynamic finger splint, worn on a finger;
Figure 1B shows a palmar view of the prior art dynamic finger splint of Fig. 1A, worn on a finger;
Figure 1C shows a perspective side view of the prior art dynamic finger splint of Figs. 1A and 1B, when not worn on a finger;
Figure 2 shows a perspective side view of a finger splint according to the invention, worn on a finger;
Figure 3 shows a side view of the finger splint of Figure 2, when not worn on a finger;
Figure 4 shows a perspective dorsal view of the finger splint of Figures 2 and 3, when not worn on a finger; and
Figure 5 shows another perspective dorsal view of an alternative finger splint according to the invention, when not worn on a finger.

### Description of embodiments

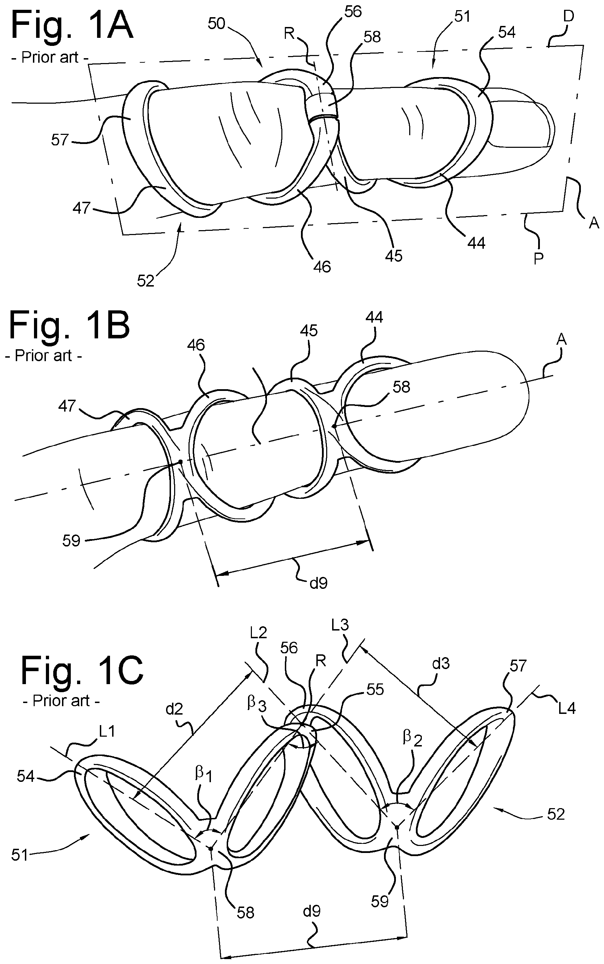

Figures 1A, 1B and 1C respectively show a dorsal and palmar view of a prior art dynamic finger splint 50 when worn on a finger and a perspective side view of the same finger splint 50 when not worn on a finger. For clarity it should be noted that "dynamic" finger splints refers to the property wherein the finger splint allows flexion of the finger to some degree, such that the finger may still be bent inwards towards the palm. The finger splint 50 is configured to receive one of a patient's index "I", middle "M", ring "R" or little finger "P".

As shown in Figures 1A and 1B, the finger splint 50 having a distal ring splint 51, for preventing overstretching of a distal interphalangeal joint (DIP) of a finger, and a proximal ring splint 52, for preventing overstretching of a proximal interphalangeal joint (PIP) of the finger, which are hingeably connected to one another, for allowing the finger being flexed. The distal splint 51 has a first distal ring 44 and a first proximal ring 45. Both rings are rigid oval rings, respectively sized to fit around the distal phalanx and the intermediate phalanx of the finger, with a first end of a longitudinal axis L1; L2 of the oval ring forming respective first and second dorsal supports 55, 54 for supporting on a dorsal side D of the intermediate and distal phalanges, at the approximate longitudinal centres of said phalanges, and an opposite second end of the longitudinal axis L1; L2 being located at the palmar side P of the DIP. The second ends of the first proximal and distal rings 44, 45 are fixedly connected to one another through a palmar connection 58, under an angle *β*2 of approximately 90°, thereby forming a rigid ring structure which allows the DIP being flexed over the palmar connection 58 while limiting the stretched position of the DIP to a normally extended position, i.e. the phalanges joined by the joint being at approximately 0° with respect to one another. The distal ring splint 51, is substantially symmetrical around a symmetry plane A which extends through both longitudinal axes L1, L2 of the first distal and proximal rings 54, 55 and is V-shaped when viewed in the symmetry plane A. A band 58 is rigidly attached to the first dorsal support 55, on a surface thereof which does not contact the finger when being worn. The band 58 is positioned substantially symmetrically around the symmetry plane A and defines an axis of rotation R perpendicular thereto.

The proximal finger splint 52 has second distal ring 46 and second proximal ring 47, which are shaped similarly as the distal finger splint, but fitting the intermediate and proximal phalanx of the finger, being connected to one another at the palmar side P of the PIP through proximal palmar connection 59, under angle *β*1 of approximately 90°. The second proximal ring 47 is provided with a fourth support 57 for being supported on the approximate centre of the dorsal side of the proximal phalanx. The second distal ring is sized to be slightly longer along a longitudinal axis L3 thereof than required to fit around the finger from the approximate centre of the intermediate phalanx on the dorsal side to the PIP on the palmar side, such that a third dorsal support 56 provided at a first end of the longitudinal axis L3, when both the distal and proximal ring splints are fitten onto the same finger, is supported on the first dorsal support 55. Hereto, when measuring the circumference of the finger for sizing of the second distal ring 46, approximately 3mm is added to the circumference measured at the approximate centre of the intermediate phalanx.

The proximal ring splint 52 and the distal ring splint 51 are each formed of resilient metal wire-like material, which is sufficiently rigid not to deform under normal movement of the finger and stop abnormal movement of the finger such as over-stretching. For comfort reasons, the longitudinal ends of each ring may be flattened to form a surface matching the finger in the respective location the longitudinal end of the ring abuts the finger during use. The dorsal supports are usually between 2.5 and 9 mm wide, measured along the length of the finger.

The band 58 of the first dorsal support is looped around the third dorsal support 56, having a size which holds the third dorsal support 56 closely onto the first dorsal support 55. The band is of a metallic material matching to the metal wire-like material of the ring splints 51, 52. Due to the stacked position of the first and third dorsal supports 55, 56, the DIP can be flexed together with the PIP, the band 58 allowing rotation of the V-shape of the distal ring splint 52 with respect to the V-shape of the proximal ring splint 51, while the stretch of the DIP with respect to the PIP is limited by surfaces of the first and third dorsal supports 55, 56 abutting each other. However, once the band 58 starts to wear, the third dorsal support 56 is no longer tightly held onto the first dorsal support 55, allowing some degree of overstretch of the finger again.

A further downside is that a distance d9 between the distal and proximal palmar connections 58, 59 is directly dependent on the length d2, d3 of the first proximal ring 45 and the second distal ring 46 along their respective longitudinal axes L2, L3 and an angle *β*3 there between at the axis of rotation R and vice versa is a maximum flexion, i,e, decrease of angle *β*3, directly dependent on the length d2, d3 of the first proximal ring 45 and the second distal ring 46 along their respective longitudinal axes L2, L3. As a result of the first, the distance d9 may not correspond to the length of the intermediate phalanx during flexing and stretching movements of the finger, causing one or both of the ring splints 51, 52 to slide with respect to the DIP and/or PIP and resulting in limited use of the joint and/or discomfort. Manufacturing and/or adjusting a finger splint such that no sliding occurs with this system is difficult, if not impossible depending on a specific patients finger anatomy. As a result of the second, the intermediate phalanx automatically limits the flexing allowed when wearing the splint. Practice has shown that the finger splint according to the prior art limits flexing of a finger to around 25% of a regular flexing motion.

Based on these above-mentioned shortcomings, there is a need for a dynamic finger splint for preventing over-stretching of both the PIP and the DIP, that is more durable and easier to make fitting to a patient's finger such that it is more comfortable to wear.

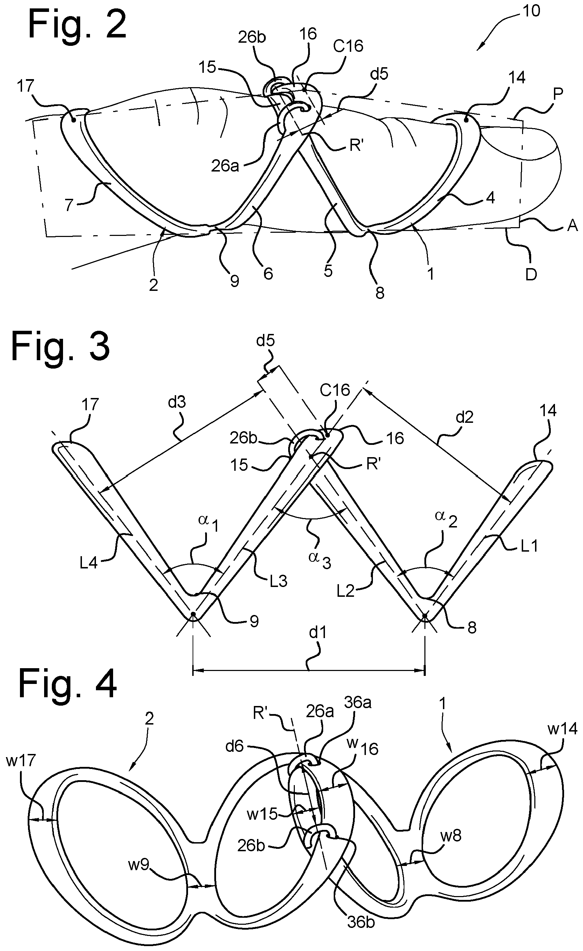

Figures 2, 3 and 4 respectively show a perspective side view of a finger splint according to the invention, worn on a finger and a side and a perspective dorsal view thereof, when not worn on a finger. The finger splint according to the invention has a rigid distal ring splint 1 and a rigid proximal ring splint 2, which are sized and shaped similar to the distal and proximal ring splints of the prior art finger splint, but are connected to one another differently.

The distal ring splint 1 thus has a first distal ring 4 and a first proximal ring 5, which both are substantially oval shaped to fit around the distal and intermediate phalanges with a first end along the longitudinal axes L1, L2 thereof forming first and second dorsal supports 14, 15 for respectively being supported on an approximate longitudinal centre of the distal and intermediate phalanx and a second end along the longitudinal axes L1, L2 being interconnected at an angle *α*2 of approximately 90° , forming a first rigid palmar connection 8 for abutting the DIP on the palmar side.

The proximal ring splint 2 has a second distal ring 6 and a second proximal ring 7, which both are substantially oval shaped to fit around the intermediate and proximal phalanges with a first end along the longitudinal axes L3, L4 thereof forming third and fourth dorsal supports 16, 17 for respectively being supported on the first dorsal support 15 and on an approximate longitudinal centre of the proximal phalanx and a second end along the longitudinal axes L3, L4 being interconnected at an angle *α*1 of approximately 90° , forming a second rigid palmar connection 9 for abutting the DIP on the palmar side. The first, second and fourth dorsal supports 15, 14, 17 each have a width W15, W14, W17 between 2 and 10 mm, seen along the length direction of the finger along the dorsal surface. The third dorsal support 16 has a width W16, which may be equal to or up to 5 mm larger than the width of the first dorsal support 15. A width W8, W9 of each of the distal and proximal palmar connections 8,9 is equal or smaller than a width of the first dorsal support 15, so as not to hinder flexing of the adjacent PIP or DIP when being worn.

The finger splint 10 according to an embodiment of the invention is provided with two rigid loops 26a, 26b, which are rigidly attached to the first distal support 15 and are spaced apart with a predetermined distance d6, substantially symmetrically with respect to a symmetry plane along the longitudinal axis L2 of the first proximal ring 5 and defining an axis of rotation R' perpendicular there to. The third dorsal support 16 is provided with two matching through-holes 36a, 36b, spaced apart with the same predetermined distance d6 and substantially symmetrical with respect to a symmetry plane along the longitudinal axis L3 of the second distal ring 6. Each of the respective loops 26a, 26b, encloses a part of a circumference of the third dorsal support 16 and is looped through one of the matching through-holes 36a, 36b to form a closed loop, thereby forming a hinging connection between the first and second ring splints 1, 2. Each hinging loop 26a, 26b has an inner diameter matching a cross-section through the third dorsal support 16, delimited by a respective through-hole 36a, 36b, such that the loops are relatively small. The hinging connection allows the proximal ring splint 2 being rotated around axis of rotation R' between a stretched position, in which a finger-facing surface section of the third dorsal support 16 abuts a surface section of the first dorsal support 15 opposite from the finger-facing surface and wherein a distance d1 between the distal and proximal palmar connections 8,9 is substantially equal to the length of the intermediate phalanx, and a flexed position, in which said distance d1 is smaller. During rotation around axis of rotation R', an inner surface of a respective through-hole 36a, 36b slides along a section of each of the loop 26a, 26b. The predetermined distance d6 between the loops 26a, 26b, ensures that the loops are not positioned directly at the position where the finger-facing surface section of the third dorsal support 16 abuts the surface section of the first dorsal support 15 opposite from the finger-facing surface, blocking further rotation to an over-stretched position. Thus in the blocked position, no additional forces are exerted directly onto the loops 26a, 26b, reducing the risk of wear and deformation thereof.

Furthermore, the predetermined distance d6 between the loops 26a, 26b, requires that the loops are attached to the second distal ring on opposite sides from the end of the longitudinal axis L2, where the ring curves downwards from said end. As a result, a distance d2 between the axis of rotation R' and the distal palmar connection 8 is smaller than if the loops were located at the longitudinal end in the symmetry plane A.

Similarly, due to the predetermined distance d6 in between the loops 26a, 26b and in between the matching through-holes 36a, 36b, a distance d5 is achieved between a central point C16 of the third dorsal support and the axis of rotation R', when seen along the longitudinal axis L3. A distance d3 between the axis of rotation R' and the proximal palmar connection 9 is decreased by the same distance d5. The predetermined distance d6 is determined such that the distances d2, d3 between the axis of rotation R' and the respective distal and proximal palmar connections 8,9 result in the distance d1 between the first and second connections 8,9 closely corresponding to the distance between the palmar side of the PIP and DIP both in the stretched position and in a flexed position of approximately 75% finger flex. In the embodiment as depicted in Figs. 2 - 4, the positioning of the through-holes 36a, 36b along the width W16 of the third dorsal support 16 may additionally be used to optimise the position of the axis of rotation R' for achieving the optimum ratio between the distance d3 to the proximal palmar connection 9 and the distance d2 to the distal palmar connection 8. The predetermined distance d6 and the positioning of the through-holes in the third dorsal support 16 in the dorsal connection according to the invention thus provide additional variables to produce a dynamic finger splint 10 having hinging properties closer matching the flexing and stretching motions of a patient's finger.

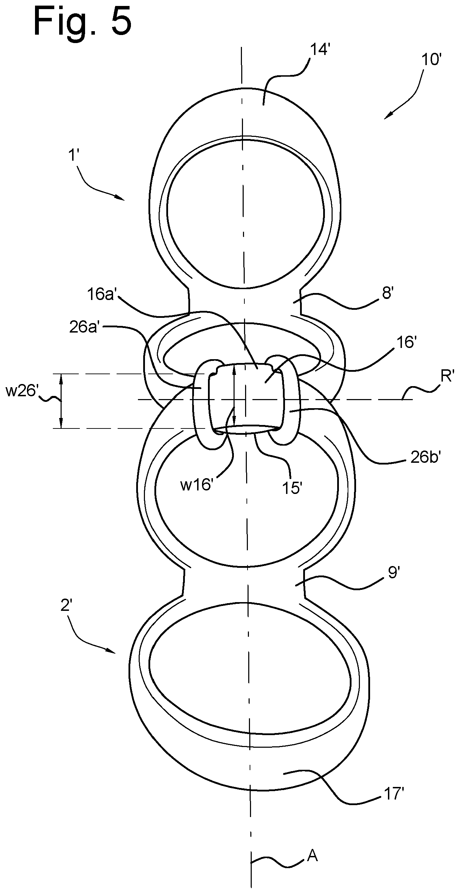

Figure 5 shows another perspective dorsal view of an alternative finger splint 10' according to the invention, when not worn on a finger. The finger splint 10' differs from the finger splint shown in Figs. 2 - 4 only in that the loops 26a', 26b' forming the dorsal connector enclose substantially the whole circumference of the third dorsal support 16', and that a section of the third dorsal support 16' in between the loops 26a', 26b' is provided with an extension 16a' such that a width W16' of the third dorsal support 16' along the symmetry plane A is larger than an inner diameter W26 of each of the loops 26a', 26b'. The extension 16a' is an optional feature which ensures that the proximal ring splint 2' may only rotate with respect to the distal ring splint 1' around the axis of rotation R'.

The present invention has been described above with reference to a number of exemplary embodiments as shown in the drawings. It is understood that modifications and alternative implementations of some parts or elements are possible and are included in the scope of protection as defined in the claims set forth hereunder.

## Claims

1. A dynamic finger splint (10; 10') for preventing over-stretching of both a proximal interphalangeal joint (PIP) and a distal interphalangeal joint (DIP) of a finger, said splint comprising:
• a rigid distal ring splint (1; 1') which comprises
- a first proximal ring (5; 5'), sized to fit around an intermediate phalanx of the finger, having a first dorsal support (15; 15') for abutting an approximate longitudinal centre of the intermediate phalanx on a dorsal side (D) with an inner surface and a section opposite thereof for abutting the intermediate phalanx at the distal interphalangeal joint on a palmar side (P) with an inner surface,
- a first distal ring (4; 4'), sized to fit around a distal phalanx of the finger, having a second dorsal support (14; 14') for abutting an approximate longitudinal centre of the distal phalanx on the dorsal side (D) with an inner surface and a section opposite thereof for abutting the distal phalanx at the distal interphalangeal joint on a palmar side (P) with an inner surface, and
- a rigid distal palmar connection (8; 8'), connecting the two sections of the first proximal and distal rings (5, 4; 5', 4'), for abutting at the distal interphalangeal joint, at an angle (α2) with respect to one another and having a width (W8), such that, when worn, the distal interphalangeal joint can be bent over the distal palmar connection;
• a rigid proximal ring splint (2; 2') which comprises
- a second distal ring (6; 6'), sized to fit around the intermediate phalanx of the finger, having a third dorsal support (16; 16'), adjoining the first proximal ring (5; 5') on the dorsal side (D) and a section opposite thereof for abutting the intermediate phalanx at the proximal interphalangeal joint on a palmar side (P) with an inner surface,
- a second proximal ring (7; 7'), sized to fit around the proximal phalanx of the finger, having a fourth dorsal support (17; 17') for abutting an approximate longitudinal centre of the proximal phalanx on the dorsal side (D) with an inner surface and a section opposite thereof for abutting the proximal phalanx at the proximal interphalangeal joint on a palmar side (P),
- a rigid proximal palmar connection (9; 9'), connecting the two sections of the second proximal and distal rings for abutting the proximal interphalangeal joint, at an angle (α1) with respect to one another and having a cross-section, such that, when worn, the proximal interphalangeal joint can be bent over the proximal palmar connection; and
• a dorsal connector, hingeably connecting the proximal ring splint (2; 2') to the distal ring splint (1; 1') on the dorsal side (D), defining an axis of rotation (R') substantially perpendicular to a symmetry plane (A) of the finger splint
wherein,
the dorsal connector is formed by two loops (26a, 26b; 26a', 26b'), which are attached to the first dorsal support (15; 15'), each loop being positioned on an opposite side of said symmetry plane (A), and forming a closed loop around at least a part of a circumference of the third dorsal support (16; 16'), each loop (26a, 26b; 26a', 26b') having an inner diameter substantially corresponding to a width of the at least part of the circumference of the third dorsal support (16; 16').

2. The dynamic finger splint (10) according to claim 1, wherein the third dorsal support (16) is provided with two through-holes (36a, 36b) on opposite sides from the symmetry plane (A), each through-hole (36a; 36b) having a loop (26a; 26b) passing therethrough.

3. The dynamic finger splint (10; 10') according to claim 1 or 2, wherein a width (W16; W16') of the circumference of the third dorsal support (16' 16') along the symmetry plane (A) is larger than the inner diameter (W26) of each loop (26a, 26b; 26a', 26b').

4. The dynamic finger splint (10; 10') according to any one of the preceding claims, wherein the dorsal connector is adapted to enable a hinging movement between a stretched position, in which an axis through the second and fourth dorsal supports (14, 17; 14', 17') along the symmetry plane (A) extends through the first dorsal support (15; 15'), and a flexed position, in which a distance (d1) between the proximal and distal palmar connections (8, 9; 8', 9') is smallerthan in the stretched position.

5. The dynamic finger splint (10; 10') according to any one of the preceding claims, wherein the angle (α1) between the proximal and distal rings (6,7; 6', 7') at the proximal palmar connection (8; 8') is between 85 and 95 degrees, preferably 90 degrees and wherein the angle (α2) between the proximal and distal rings (4, 5; 4', 5') at the distal palmar connection (9; 9') is between 40 and 85 degrees, preferably between 55 and 65 degrees.

6. The dynamic finger splint (10; 10') according to any one of the preceding claims, wherein a distance between the second palmar connection (9; 9') and a centre (C16) of the third dorsal support (16; 16') is larger than a distance between the second palmar connection (9; 9') and the axis of rotation (R').

7. The dynamic finger splint (10; 10') according to any one of the preceding claims, wherein the first, second and fourth dorsal support (14,15,17; 14',15',17') each have a width (W14, W15, W17) along the symmetry plane (A) between 2 and 9 mm, preferably between 2.5 and 8 mm.

8. The dynamic finger splint (10; 10') according to any one of the preceding claims, the finger splint being made of one or more metallic materials, preferably at least one of a silver alloy, a gold alloy, a titanium alloy or stainless steel.

9. A method of manufacturing a dynamic finger splint (10; 10') according to the preamble of claim 1, said method comprising:
- obtaining a patient's finger measurements, determining a length and a circumference of each of the proximal, intermediate and distal phalanges,
- producing according to those measurements the rigid distal ring splint (1; 1'), for preventing over-stretch of a distal interphalangeal joint of the finger, and the rigid proximal ring splint (2; 2'), for preventing over-stretch of a proximal interphalangeal joint of the finger, the rigid distal ring splint (1; 1'),
- joining the proximal ring splint (2; 2') to the distal ring splint (1; 1') by providing two loops (26a, 26b; 26a', 26b'), each having an inner diameter (W26; W26') equal to or smaller than a width (W16; W16') of the third dorsal support (16; 16') along the symmetry plane (A) and closing each loop around at least part of the circumference of the third dorsal support (16; 16').

10. The method according to claim 9, wherein the step of joining further comprises providing two through-holes in the third dorsal support (16; 16'), which are spaced apart on opposite sides from the symmetry plane (A) having a distance there between matching a distance (d6) between the two protrusions for forming loops.

11. The method according to claim 9 or 10, wherein the producing of the rigid distal ring splint (1; 1') comprises providing two protrusion for forming loops, which are attached to the first dorsal support (15; 15'), spaced apart on opposite sides from the symmetry plane (A), and wherein the step of joining the proximal ring splint (2; 2') to the distal ring splint (1; 1') comprises shaping each of the two protrusions into a loop (26a, 26b; 26a', 26b') having an inner diameter (W26; W26') equal to or smaller than a width (W16; W16') of the third dorsal support (16; 16') along the symmetry plane (A).

12. The method according to any one of claims 9 to 11, wherein the step of producing the rigid distal and proximal ring splints comprises:
- producing a wax-mould of the rigid distal ring splint and of the proximal ring splint according to the patient's finger measurements, and
- casting the rigid distal ring splint and the rigid proximal ring splint from a metallic material.

13. The method according to any one of claims 9 to 11, wherein the step of producing the rigid distal and proximal ring splints comprises three-dimensional printing of the rigid distal and proximal ring splints from a metallic material.

14. The method according to claim 12 or 13, wherein the metallic material comprises at least one of silver, gold, bronze or titanium.

15. The method according to any one of claims 9 to 14, wherein the closing of each loop comprises soldering the loop to be closed.
